# EUROPÄISCHE PATENTSCHRIFT

(11) **EP 4 193 971 B1**
(45) Veröffentlichungstag und Bekanntmachung des Hinweises auf die Patenterteilung: **28.10.2026**
(21) Anmeldenummer: 22212692.2
(22) Anmeldetag: 12.12.2022
(51) Int. Cl.: A61F 5/01, A61F 5/30

(54) **VERFAHREN ZUR HERSTELLUNG EINER ORTHESE**
METHOD FOR PRODUCING AN ORTHOSIS
PROCÉDÉ DE FABRICATION D'UNE ORTHÈSE

(30) Priorität: 13.12.2021 DE 102021132820
(43) Veröffentlichungstag der Anmeldung: 14.06.2023
(73) Patentinhaber: Hoffmann, Marco, 34132 Kassel (DE); Biller, Stefan, 40549 Düsseldorf (DE); Morth, Stephan, 37213 Witzenhausen (DE)
(72) Erfinder: Hoffmann, Marco, 34132 Kassel (DE); Biller, Stefan, 40549 Düsseldorf (DE); Morth, Stephan, 37213 Witzenhausen (DE)
(74) Vertreter: Hannke Bittner & Partner mbB Regensburg

(56) Entgegenhaltungen:
- DE-A1- 102019 109 781
- US-A- 4 428 369
- US-A- 5 807 294
- US-A1- 2015 328 016

## Beschreibung

Die Erfindung betrifft ein Verfahren zur Herstellung einer Orthese.

Eine Orthese ist ein medizinisches Hilfsmittel, welches als äußerlich an ein Körperteil angelegtes Gerät zur Beeinflussung der strukturellen und funktionellen Eigenschaften des neuromuskulären und skelettalen Systems eines Menschen ausgebildet ist. Nach einem internationalen Klassifikationssystem werden Orthesen in Klassen von vier Körperbereichen zusammengefasst. Zum einen gibt es Orthesen der unteren Extremitäten, zum anderen Orthesen der oberen Extremitäten. Weiterhin gibt es Orthesen für den Rumpf und Orthesen für den Kopf.

Bei der traditionellen Herstellung von Orthesen wird von dem zu versorgenden Körperteil ein Abbild - oft ein Gipsabdruck - in korrigierter Stellung und Form des Körperteils erstellt. Aus dem Abbild wird handwerklich durch Auf- und Abtragen von Material, Anbringen von Bauteilen und Platzhaltern und weiteren Arbeitsschritten die Zweckform als Positivform des zu versorgenden Körperteils zur Herstellung der Orthese gefertigt. Die äußere Form der Zweckform entspricht dabei weitgehend der Innenform der Orthese. Anschließend wird bei der traditionellen Herstellung die Orthese durch Tiefziehen über die Zweckform, Umgießen der Zweckform oder ähnliche Herstellungsmethoden erstellt. In zusätzlichen Arbeitsschritten wird die Orthese danach weiter an den Patienten beziehungsweise dessen zu versorgenden Körperteils angepasst. Bei diesem traditionellen handwerklichen Ablauf der Orthesenherstellung werden Polster ganzflächig oder lokal auf die Zweckform aufgebracht. Während dieses handwerklichen Fertigungsprozesses der Orthese wird die Position und die Form des Polstermaterials in der Orthese berücksichtigt. Die Polster werden anschließend von der Zweckform entfernt und fest mit der Orthese verklebt.

Neben der handwerklichen Herstellung von Orthesen hat sich in den letzten Jahren auch ein digitaler Herstellungsprozess für Orthesen etabliert. Dabei wird in der Regel ein digitales Abbild des zu versorgenden Körperteils, der Zweckform und der Orthese erstellt. Ein derartiges Herstellungsverfahren ist beispielsweise in der WO 2019 129419 A1 beschrieben. Allerdings gibt es im digitalen Herstellungsprozess kein physisches Positivmodell des Körperteils mehr, sodass entsprechende Polster per Augenmaß und mit höherem Aufwand nachträglich zugeschnitten in die Orthese geformt werden müssen. Insofern muss auch bei digital erstellten Orthesen während der Herstellung Freiraum für Polstermaterial vorgesehen werden. Dieser Freiraum wird in der Regel entweder durch die Form der digitalen Zweckform oder durch Abweichungen der digitalen Orthese zur digitalen Zweckform berücksichtigt. Durch den digitalen Ablauf muss das einzubringende reale Polster getrennt von der Orthese und Zweckform hergestellt und anschließend in die gefertigte Orthese eingefügt beziehungsweise eingeklebt werden. Dabei ist die, durch den digital berücksichtigten Freiraum, geplante Form und Position des Polsters in der Regel nicht mehr exakt nachvollziehbar.

Ein ähnliches Verfahren ist auch aus der DE 10 2019 109 781 A1 bekannt, bei der anhand eines vorgegebenen Polsterbereichs einer digitalen Orthese, in die daraus hergestellte Orthese später ein Polster eingesetzt wird.

Derartige Polster beziehungsweise auch Polsterträger sind beispielsweise auch aus den Druckschriften US 5 807 294 A, US 4 428 369 A sowie US 2015/328016 A1 bekannt. Allerdings ergeben sich bei Verwendung derartiger Polster beziehungsweise Polsterträger in digital hergestellten Orthesen die bereits eingangs beschriebenen Probleme der separaten Herstellung gegenüber der Orthese und Zweckform, wobei die, durch den digital berücksichtigten Freiraum, geplante Form und Position des Polsters in der Regel nicht mehr exakt nachvollziehbar ist.

Der Erfindung liegt daher die Aufgabe zugrunde, ein Verfahren zur Herstellung einer Orthese bereitzustellen, bei der auch im digitalen Herstellungsprozess Polster exakt geformt und exakt in der Orthese positioniert werden können.

Gelöst wird diese Aufgabe verfahrensgemäß durch ein Verfahren mit allen Merkmalen des Patentanspruchs 1. Vorteilhafte Ausgestaltungen der Erfindung finden sich in den Unteransprüchen.

Das erfindungsgemäße Verfahren zur Herstellung einer Orthese weist folgende Schritte auf.
a) Erstellen eines digitalen Abbildes eines zu versorgenden Körperteils,
b) Erstellen einer digitalen Zweckform des zu versorgenden Körperteils anhand des digitalen Abbildes,
c) Markieren wenigstens eines digitalen Polsterbereichs der digitalen Zweckform,
d) Erstellen einer digitalen Orthese anhand der digitalen Zweckform und wenigstens einem digitalen Polsterbereichs,
e) Erstellen einer realen Orthese anhand der digitalen Orthese und zeichnet sich dadurch aus, dass in Schritt d) zusätzlich anhand der digitalen Zweckform und des wenigstens einen digitalen Polsterbereichs wenigstens ein digitaler Polsterträger erstellt wird, und
in Schritt e) zusätzlich anhand der digitalen Orthese und des digitalen Polsterträgers wenigstens ein realer Polsterträger erstellt wird und abschließend ein Fügen des wenigstens einen realen Polsterträgers mit der realen Orthese stattfindet, wobei der reale Polsterträger in seinen Ausmaßen begrenzt ist auf den Bereich, in dem Polstermaterial vorgesehen ist und wobei das Erstellen einer digitalen Orthese und wenigstens eines digitalen Polsterträgers derart erfolgt, dass die digitale Orthese wenigstens ein digitales Positionierungselement und der wenigstens eine digitale Polsterträger ein dazu passendes digitales Gegenpositionierungselement aufweist, sodass die reale Orthese wenigstens ein reales Positionierungselement für den wenigstens einen realen Polsterträger und der wenigstens eine reale Polsterträger ein dazu passendes reales Gegenpositionierungselement aufweist.

Bei dem erfindungsgemäßen Verfahren wird zu Beginn ein digitales Abbild eines zu versorgenden Körperteils, vorzugsweise in seiner korrigierten Stellung, erstellt. Nachfolgend wird anhand dieses digitalen Abbildes eine digitale Zweckform des zu versorgenden Körperteils erstellt. Die nachfolgenden erfindungswesentlichen Schritte bestehen nun darin, dass an der digitalen Zweckform nunmehr wenigstens ein digitaler Polsterbereich markiert wird, anhand dessen nunmehr neben der Erstellung einer digitalen Orthese nunmehr auch ein digitaler Polsterträger erstellt werden kann. Während der Erstellung der digitalen Orthese wird der für die Polster berücksichtigte Raum dabei auf der Innenseite der Orthese durch ein extra anzufertigendes Bauteil in Form des Polsterträgers abgebildet beziehungsweise festgehalten. Die Form des Polsterträgers entspricht beispielsweise der Innenform oder der Außenform der Orthese und ist in seinen Ausmaßen begrenzt auf den Bereich, in dem Polstermaterial vorgesehen ist, wobei das Polstermaterial auch etwas über den Polsterträger hinausragen kann, um Druckstellen vorzubeugen oder Stoßkanten zu vermeiden; andererseits kann das Polstermaterial gegenüber dem Polsterträger auch zurückgeschnitten sein. Das Bauteil wird getrennt von der Orthese gefertigt und dient durch seine Form als Schablone zum Zuschnitt von Polstermaterial und zur Formgebung des Polstermaterials. Anhand der digitalen Orthese und des digitalen Polsterträgers kann dann nachfolgend eine reale Orthese mit wenigstens einem realen Polsterträger erstellt werden. In einem abschließenden Schritt werden die reale Orthese mit dem wenigstens einen realen Polsterträger gefügt. Vor dem Fügen oder auch danach kann der reale Polsterträger mit entsprechenden Polstern beaufschlagt werden. Durch das erfindungsgemäße Verfahren ist es nunmehr ermöglicht, während des digitalen Herstellungsverfahrens bereits Polsterbereiche zu berücksichtigen und separate Polsterträger digital zu erstellen, sodass eine nach dem Fügen der Orthese mit dem Polsterträger kein nachträgliches Bearbeiten der Orthese zum Aufbringen von Polstern innerhalb der Orthese mehr notwendig ist.

Durch diese Ausgestaltung des erfindungsgemäßen Herstellungsverfahrens ist sichergestellt, dass die reale Orthese und das reale Positionierungselement exakt miteinander gefügt werden können, sodass das zu versorgende Körperteil durch die reale Orthese samt realem Polsterträger exakt in der notwendigen Stellung gehalten wird, ohne dass eine nachträgliche Bearbeitung der realen Orthese oder des realen Polsterträgers vor dem endgültigen Fügen dieser Teile, nochmals aufwendig bearbeitet werden müssen. Ein nachträgliches Bearbeiten der Orthese zum Anbringen des Polsterträgers, beispielsweise mittels Aufbringens von Klebstoff oder dergleichen, wird daher durch das erfindungsgemäße Verfahren obsolet.

Nach einer besonders vorteilhaften Ausgestaltung des erfindungsgemäßen Verfahrens erfolgt ein lösbares Fügen des wenigstens einen realen Polsterträgers an der realen Orthese durch das Positionierungselement und das Gegenpositionierungselement. Hierdurch ist es ermöglicht, dass verschiedene Polsterträger, beispielsweise mit verschiedenen Polsterungen hergestellt werden können, die z. B. an bestimmte Aktivitäten des Trägers der Orthese angepasst sind. Zudem ist es möglich, im Bereich des Polsterträgers einfachere Änderungen vorzunehmen, sodass nicht gerade die gesamte Orthese aufwendig nachgearbeitet werden muss.

Eine weiterhin vorteilhafte Ausgestaltung des erfindungsgemäßen Verfahrens besteht darin, dass das Fügen des wenigstens einen realen Polsterträgers mit der realen Orthese durch das Positionierungselement und das Gegenpositionierungselement derart erfolgt, dass der wenigstens eine reale Polsterträger an der Orthese formschlüssig, insbesondere unverschiebbar und/oder unverdrehbar fixierbar ist. Durch diese Ausgestaltung des erfindungsgemäßen Verfahrens ist erreicht, dass der Polsterträger derart in der Orthese fixiert werden kann, dass ein Verschieben und/oder Verdrehen des Polsterträgers innerhalb der Orthese nicht möglich ist, sodass die exakte Fixierung dauerhaft aufrechterhalten werden kann. Hierdurch ist gewährleistet, dass das zu versorgende Körperteil auf Dauer exakt in korrigierter Stellung gehalten werden kann und somit die erwünschten therapeutischen Effekte durch das Tragen der Orthese erreicht werden und eine Fehlstellung aufgrund eines sich verdrehenden beziehungsweise verschiebenden Polsterträgers nicht auftreten kann.

Dabei kann es auch vorgesehen sein, dass Positionierungs- und Gegenpositionierungselemente in Form einer lösbaren Clips- beziehungsweise Rastverbindung miteinander gefügt werden. Eine derartige Verbindung bietet den Vorteil, dass der Polsterträger sicher und unverlierbar in der Orthese gehalten ist, auch wenn die Orthese vom Benutzer nicht getragen wird.

Nach einer weiteren Ausgestaltung des erfindungsgemäßen Verfahrens ist es vorgesehen, dass der Polsterträger auf seiner zum Körperteil hinweisenden Seite mit einem Polstermaterial versehen wird. Dieses Polstermaterial dient dazu, dass ein angenehmes Tragen der Orthese ermöglicht ist, ohne dass im Bereich des Polsterträgers Beschwerden, wie Rötungen, Wundsein oder dergleichen auftreten.

Nach einer anderen Ausgestaltung des erfindungsgemäßen Verfahrens ist es vorgesehen, dass der Polsterträger auf seiner zum Körperteil wegweisenden Seite mit einem Polstermaterial versehen wird.

Bei vielen Orthesen, beispielsweis im Bereich der Extremitäten, wird der separate Polsterträger zwischen Körperteil und Orthese angeordnet sein. Es gibt aber auch Orthesen, beispielsweise im Kopfbereich, vom beispielsweise zum Schutz Operationsöffnungen, bei denen es sich anbietet, die Orthese zwischen dem Körperteil und dem Polsterträger anzuordnen, damit der Polsterträger als Stoßschutz dienen kann.

Dabei hat es sich bewährt, dass der Polsterträger samt Polstermaterial als Polsterverbund beispielsweise in Sandwichbauweise hergestellt wird. Alternativ ist es möglich, dass der Polsterträger selbst bereits aus einem Polstermaterial besteht, sodass auf ein zusätzliches beziehungsweise separates Polstermaterial verzichtet werden kann.

Die Orthese zeichnet sich dadurch aus, dass sie wenigstens einen separaten Polsterträger aufweist. Vorteilhaft bei der Orthese ist es, dass der Polsterträger als separates Bauteil der Orthese hergestellt werden kann und somit auch unterschiedliche Polsterträger mit unterschiedlichen Polstern hergestellt werden können, sodass ein separater Austausch einzelner Polsterträger ermöglicht ist.

Dabei hat es sich weiterhin als vorteilhaft erwiesen, dass die Orthese mit den wenigstens einen Polsterträger lösbar gefügt ist. Hierdurch ist es in einfacher Weise ermöglicht, das Polsterträger ohne Probleme und ohne größeren Aufwand ausgetauscht werden können.

Dabei hat es sich als äußerst vorteilhaft erwiesen, dass die Orthese wenigstens ein Positionierungselement aufweist, in welches ein entsprechendes Gegenpositionierungselement des Polsterträgers eingreift. Hierdurch ist ein einfaches Verbinden des Polsterträgers mit der Orthese und auch ein wieder Lösen in einfacher Weise ermöglicht.

Nach einem weiteren vorteilhaften Gedanken sind die Orthese und der wenigstens eine Polsterträger unverdrehbar und/oder unverschiebbar miteinander gefügt. Durch ein derartiges Fügen wird ein formschlüssiges Verbinden des Polsterträgers mit der Orthese erreicht, welches über entsprechende Positionierungselemente und Gegenpositionierungselemente der Orthese beziehungsweise des Polsterträgers realisiert werden können. Hierdurch wird ein sicheres Fixieren des Polsterträgers in der Orthese ermöglicht, sodass das Körperteil innerhalb der Orthese in seiner korrigierten Stellung gehalten wird, ohne dass ein sich verdrehender beziehungsweise verschiebender Polsterträger sich negativ auf die korrigierte Stellung des zu versorgenden Körperteils innerhalb der Orthese auswirkt.

Nach einem weiteren Gedanken ist es vorgesehen, dass die Orthese nach einem der zuvor beschriebenen Verfahren hergestellt ist. Allerdings ist es auch möglich, dass eine nach traditionellen handwerklichen Verfahren hergestellte Orthese mit einem erfindungsgemäßen Polsterträger versehen wird. Insofern ist die erfindungsgemäße Orthese nicht nur auf ein digitales Herstellungsverfahren beschränkt, sondern kann auch traditionell hergestellt werden.

Weitere Ziele, Vorteile, Merkmale und Anwendungsmöglichkeiten der vorliegenden Erfindung ergeben sich aus der nachfolgenden Beschreibung von Ausführungsbeispielen anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Figur 1:: ein Ausführungsbeispiel eines digitalen Abbildes eines Fußes, an den eine Orthese angepasst werden soll,
- Figur 2:: ein Ausführungsbeispiel einer digitalen Zweckform des digitalen Abbildes der Figur 1,
- Figur 3: ein Ausführungsbeispiel der digitalen Zweckform der Figur 2 mit markiertem Polsterbereich,
- Figur 4: ein Ausführungsbeispiel einer digitalen Orthese samt digitalem Polsterträger angepasst an die digitale Zweckform der Figur 3,
- Figur 5: Ausführungsbeispiele der anhand der digitalen Orthese und des digitalen Polsterträgers der Figur 4 nach dem erfindungsgemäßen Verfahren hergestellten Orthese und Polsterträger,
- Figur 6: das Ausführungsbeispiel der Orthese der Figur 5 samt Polsterträger mit Polstermaterial,
- Figur 7: die Orthese gemäß Figur 6 samt mit ihr gefügtem Polsterträger,
- Figur 8: ein weiteres Ausführungsbeispiel einer nach dem erfindungsgemäßen Verfahren hergestellte Orthese samt Polsterträger mit Polstermaterial und
- Figur 9: die Orthese gemäß Figur 8 samt mit ihr gefügtem Polsterträger.

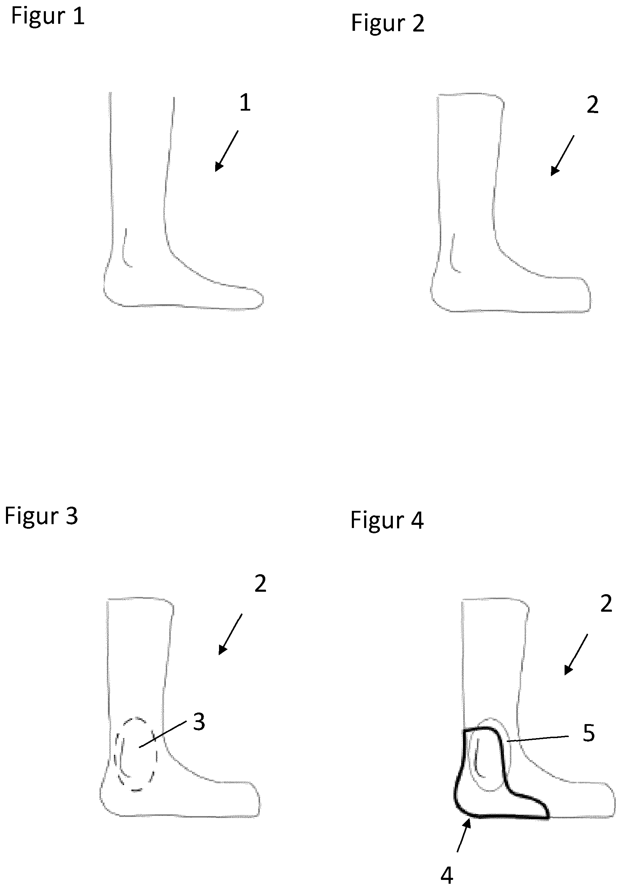

In den Figuren 1 bis 5 sind verschiedene Schritte eines erfindungsgemäßen digitalen Herstellungsverfahrens einer erfindungsgemäßen Orthese dargestellt.

Dabei zeigt Figur 1 ein digitales Abbild 1 des Körperteils, an dem die Orthese angepasst wird. Im vorliegenden Beispiel ist das Körperteil ein menschlicher Fuß.

Anhand des digitalen Abbildes 1 des Fußes wird in einem zweiten Schritt eine digitale Zweckform 2 als Positivmodell des Fußes erstellt, wie sie beispielhaft in Figur 2 dargestellt ist.

In dieser digitalen Zweckform 2 wird - wie in Figur 3 dargestellt - nunmehr ein digitaler Polsterbereich 3 markiert. Dieser digitale Polsterbereich 3 entspricht einem Polsterbereich, der zu fertigenden Orthese, wie er an dem zu versorgenden Körperteil - wie dem Fuß - angeordnet sein muss.

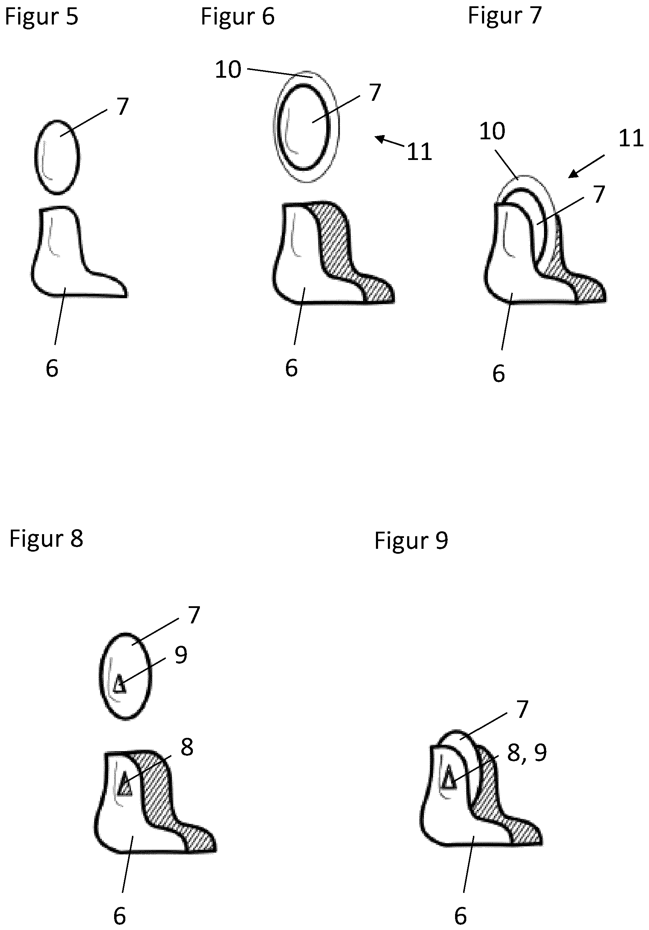

In einem weiteren digitalen Schritt wird nun eine digitale Orthese 4 samt digitalem Polsterträger 5 anhand der digitalen Zweckform 2 und dem darauf angeordneten digitalen Polsterbereich 3 erstellt. Eine entsprechende Darstellung zeigt Figur 4. Der digitale Polsterträger 5 entspricht dabei in seiner Form der Innenform einer entsprechenden Orthese ohne Polsterträger, wobei in der digitalen Orthese 4 ein entsprechender Freiraum für den digitalen Polsterträger vorgesehen wird. Anhand der digitalen Orthese 4 und des digitalen Polsterträgers 5 können nun über entsprechend bekannte Herstellungsverfahren - wie beispielsweise 3D-Druck, Spritzgussverfahren oder dergleichen - eine reale Orthese 6 und ein realer Polsterträger 7 gefertigt werden, wie sie in Figur 5 dargestellt sind.

In der Figur 6 ist nunmehr ein weiteres Ausführungsbeispiel einer erfindungsgemäßen Orthese 6 samt Polsterträger 7 gezeigt. Dabei ist der Polsterträger 7 bereits mit einem Polstermaterial 10 versehen. Der Polsterträger 7 samt Polstermaterial 10 sind dabei vorteilhaft in Sandwichbauweise hergestellt. Vorteilhafterweise kann eine solche Sandwichbauweise des Polsterverbundes als Verbundmaterial erfolgen. Natürlich ist es auch möglich, den Polsterträger 7 und das Polstermaterial 10 als separate Bauteile zu fertigen und nachfolgend miteinander zu fügen beispielsweise zu verkleben.

In Figur 7 ist nun nachfolgend der Polsterverbund 11 in Form des Polsterträgers 7 samt Polstermaterial 10 mit der Orthese 6 gefügt. Dieses Fügen kann durch unterschiedlich bekannte Fügetechniken erfolgen.

Ein besonders vorteilhaftes Fügen ist dabei in den Figuren 8 und 9 dargestellt. Wie insbesondere der Figur 8 zu entnehmen ist, weist die Orthese 6 ein Positionierungselement 8 in Form eines ausgeschnittenen Dreiecks auf, in welches ein Gegenpositionierungselement 9 in Form eines entsprechend auf den Polsterträger 7 angeordneten Dreiecks formschlüssig eingreifen kann.

Ein derartig formschlüssig mit der Orthese 6 gefügter Polsterträger 7 ist in der Figur 9 gezeigt. Dabei ist das Positionierungselement 8 der Orthese 6 in Eingriff mit dem Gegenpositionierungselement 9 des Polsterträgers 7. Durch die spezielle Ausgestaltung des Positionierungselementes 8 und des Gegenpositionierungselementes 9 in Form von entsprechenden ineinandergreifenden Dreiecken ist der Polsterträger 7 somit unverschiebbar und unverdrehbar in der Orthese 6 gehalten. Die Darstellung des Positionierungselementes 8 und des Gegenpositionierungselementes 9 in den Figuren 8 und 9 ist allerdings nur beispielhaft. Natürlich sind auch andere Positionierungselemente und Gegenpositionierungselemente denkbar, die ein Verschieben und Verdrehen des Polsterträgers 7 innerhalb der Orthese 6 verhindern.

Ferner können das Positionierungselement 8 und das Gegenpositionierungselement 9 mit entsprechenden hier nicht dargestellten Clips- oder Rastelementen versehen sein, sodass der Polsterträger 7 nicht nur verdreh- und verschiebesicher, sondern auch unverlierbar in der Orthese 6 gehalten ist. Dadurch ist auch ein sicheres, unverlierbares Halten des Polsterträgers 7 in der Orthese 6 gewährleistet, wenn die Orthese vom Benutzer nicht getragen wird.

### Bezugszeichenliste

- 1: digitales Abbild
- 2: digitale Zweckform
- 3: digitaler Polsterbereich
- 4: digitale Orthese
- 5: digitaler Polsterträger
- 6: Orthese
- 7: Polsterträger
- 8: Positionierungselement
- 9: Gegenpositionierungselement
- 10: Polstermaterial
- 11: Polsterverbund

## Patentansprüche

1. Verfahren zur Herstellung einer Orthese mit folgenden Schritten:
a) Erstellen eines digitalen Abbildes (1) eines zu versorgenden Körperteils,
b) Erstellen einer digitalen Zweckform (2) des zu versorgenden Körperteils anhand des digitalen Abbildes (1),
c) Markieren wenigstens eines digitalen Polsterbereichs (3) der digitalen Zweckform (2),
d) Erstellen einer digitalen Orthese (4) anhand der digitalen Zweckform (2) und des wenigstens einen digitalen Polsterbereichs (3),
e) Erstellen einer realen Orthese (6) anhand der digitalen Orthese (4),
**dadurch gekennzeichnet, dass**
in Schritt d) zusätzlich anhand der digitalen Zweckform (2) und des wenigstens einen digitalen Polsterbereichs (3) wenigstens ein digitaler Polsterträger (5) erstellt wird, und in Schritt e) zusätzlich anhand der digitalen Orthese (4) und des digitalen Polsterträgers (5) wenigstens ein realer Polsterträger (7) erstellt wird und abschließend ein Fügen des wenigstens einen realen Polsterträgers (7) mit der realen Orthese stattfindet, wobei der reale Polsterträger (7) in seinen Ausmaßen begrenzt ist auf den Bereich, in dem Polstermaterial vorgesehen ist und wobei das Erstellen einer digitalen Orthese (4) und wenigstens eines digitalen Polsterträgers (5) derart erfolgt, dass die digitale Orthese (4) wenigstens ein digitales Positionierungselement und der wenigstens eine digitale Polsterträger (5) ein dazu passendes digitales Gegenpositionierungselement aufweist, sodass die reale Orthese (6) wenigstens ein reales Positionierungselement (8) für den wenigstens einen realen Polsterträger (7) und der wenigstens eine reale Polsterträgers (7) ein dazu passendes reales Gegenpositionierungselement (9) aufweist.

2. Verfahren nach Anspruch 1, **dadurch gekennzeichnet, dass** ein lösbares Fügen des wenigstens einen realen Polsterträgers (7) an der realen Orthese (6) durch das Positionierungselement (8) und das Gegenpositionierungselement (9) erfolgt.

3. Verfahren nach einem der vorhergehenden Ansprüche, **dadurch gekennzeichnet, dass** das Fügen des wenigstens einen realen Polsterträgers (7) mit der realen Orthese (6) durch das Positionierungselement (8) und das Gegenpositionierungselement (9) derart erfolgt, dass der wenigstens eine reale Polsterträgers (7) an der Orthese (6) formschlüssig, insbesondere unverschiebar und/oder unverdrehbar fixierbar ist.

4. Verfahren nach einem der vorhergehenden Ansprüche, **dadurch gekennzeichnet, dass** der Polsterträger (7) auf seiner zum Körperteil hinweisenden Seite mit einem Polstermaterial (10) versehen wird.

5. Verfahren nach einem der vorhergehenden Ansprüche, **dadurch gekennzeichnet, dass** der Polsterträger (7) auf seiner zum Körperteil wegweisenden Seite mit einem Polstermaterial (10) versehen wird.

6. Verfahren nach Anspruch 4 oder 5, **dadurch gekennzeichnet, dass** der Polsterträger (7) samt Polstermaterial (10) als Polsterverbund (11) beispielsweise in Sandwichbauweise hergestellt wird.

## Claims

1. A method for producing an orthosis, comprising the following steps:
a. creating a digital model (1) of a body part to be fitted,
b. creating a digital functional form (2) of the body part to be fitted based on the digital model (1),
c. marking at least one digital padding region (3) of the digital positive model (2),
d. creating a digital orthosis (4) based on the digital positive model (2) and the at least one digital padding region (3),
e. creating a real orthosis (6) based on the digital orthosis (4),
**characterized in that**
in step d), at least one digital pad carrier (5) is additionally created on the basis of the digital positive model (2) and the at least one digital padding region (3),
and in step e), at least one real pad carrier (7) is additionally created on the basis of the digital orthosis (4) and the digital pad carrier (5), and finally the at least one real pad carrier (7) is joined to the real orthosis (6),
wherein the real pad carrier (7) is dimensionally limited to the region in which padding material is provided,
and wherein the creation of the digital orthosis (4) and the at least one digital pad carrier (5) is carried out such that the digital orthosis (4) comprises at least one digital positioning element and the at least one digital pad carrier (5) comprises a corresponding digital counter-positioning element,
so that the real orthosis (6) comprises at least one real positioning element (8) for the at least one real pad carrier (7), and the at least one real pad carrier (7) comprises a corresponding real counter-positioning element (9).

2. The method according to claim 1,
**characterized in that**
a detachable joining of the at least one real pad carrier (7) to the real orthosis (6) is effected by means of the positioning element (8) and the counter-positioning element (9).

3. The method according to any one of the preceding claims,
**characterized in that**
the joining of the at least one real pad carrier (7) to the real orthosis (6) by means of the positioning element (8) and the counter-positioning element (9) is effected such that the at least one real pad carrier (7) is positively locked to the orthosis (6), in particular fixed against displacement and/or rotation.

4. The method according to any one of the preceding claims,
**characterized in that**
the pad carrier (7) is provided with a padding material (10) on its side facing the body part.

5. The method according to any one of the preceding claims,
**characterized in that**
the pad carrier (7) is provided with a padding material (10) on its side facing away from the body part.

6. The method according to claim 4 or 5,
**characterized in that**
the pad carrier (7) together with the padding material (10) is manufactured as a padding composite (11), for example in sandwich construction.

## Revendications

1. Procédé de fabrication d'une orthèse comportant les étapes suivantes :
a) création d'une image numérique (1) d'une partie du corps à soigner ;
b) création d'un moule cible numérique (2) de la partie du corps à soigner à partir de l'image numérique (1);
c) marquage d'au moins une zone de rembourrage numérique (3) du moule cible numérique (2) ;
d) création d'une orthèse numérique (4) à partir du moule cible numérique (2) et de l'au moins une zone de rembourrage numérique (3) ;
e) création d'une orthèse réelle (6) à partir de l'orthèse numérique (4),
**caractérisé par le fait qu'**
à l'étape d), au moins un support de rembourrage numérique (5) est en outre créé à partir du moule cible numérique (2) et de l'au moins une zone de rembourrage numérique (3), et à l'étape e), au moins un support de rembourrage réel (7) est en outre créé à partir de l'orthèse numérique (4) et du support de rembourrage numérique (5), puis un assemblage de l'au moins un support de rembourrage réel (7) avec l'orthèse réelle a lieu, le support de rembourrage réel (7) étant limité, dans ses dimensions, à la zone dans laquelle un matériau de rembourrage est prévu, et la création d'une orthèse numérique (4) et d'au moins un support de rembourrage numérique (5) s'effectuant de telle sorte que l'orthèse numérique (4) présente au moins un élément de positionnement numérique et que l'au moins un support de rembourrage numérique (5) présente un élément de contre-positionnement numérique correspondant, de sorte que l'orthèse réelle (6) présente au moins un élément de positionnement réel (8) pour l'au moins un support de rembourrage réel (7) et que l'au moins un support de rembourrage réel (7) présente un élément de contre-positionnement réel (9) correspondant.

2. Procédé selon la revendication 1, **caractérisé par le fait qu'**un assemblage démontable de l'au moins un support de rembourrage réel (7) sur l'orthèse réelle (6) est réalisé par l'élément de positionnement (8) et l'élément de contre-positionnement (9).

3. Procédé selon l'une des revendications précédentes, **caractérisé par le fait que** l'assemblage de l'au moins un support de rembourrage réel (7) avec l'orthèse réelle (6) s'effectue à l'aide de l'élément de positionnement (8) et de l'élément de contre-positionnement (9) de telle sorte que l'au moins un support de rembourrage réel (7) soit apte à être fixé à l'orthèse (6) par complémentarité de forme, en particulier de manière à ne pas pouvoir coulisser et/ou pivoter.

4. Procédé selon l'une des revendications précédentes, **caractérisé par le fait que** le support de rembourrage (7) est muni, sur son côté tourné vers la partie du corps, d'un matériau de rembourrage (10).

5. Procédé selon l'une des revendications précédentes, **caractérisé par le fait que** le support de rembourrage (7) est muni d'un matériau de rembourrage (10) sur son côté opposé à la partie du corps.

6. Procédé selon la revendication 4 ou 5, **caractérisé par le fait que** le support de rembourrage (7), avec le matériau de rembourrage (10), est fabriqué sous la forme d'un composite de rembourrage (11), par exemple selon une structure en sandwich.
